# EUROPEAN PATENT APPLICATION

(11) **EP 1 002 868 A1**
(43) Date of publication of application: **24.05.2000**
(21) Application number: 98830674.2
(22) Date of filing: 09.11.1998
(51) Int. Cl.: C12N 15/74, C12N 15/31, A01N 63/00

(54) **Method to control gene expression in bacteria namely rhyzobiaceae to improve root nodule development nitrogen fixation and plant biomass production**

(71) Applicant: G.IN.E.ST.R.A. Società Consortile a.r.l., 37100 Verona (IT); CONSIGLIO NAZIONALE DELLE RICERCHE, 00185 Roma (IT)
(72) Inventor: Defez, Roberto, 80125 Napoli (IT); Spena, Angelo, 37100 Verona (IT)
(74) Representative: de Simone, Domenico

(57) **Abstract**

A promintron sequence derived from an intervening sequence of the *rolA* gene of *Agrobacterium rhizogenes* strain A4 is described. The sequence is able to drive gene expression within bacteroids in all stages of nodule development in order to obtain, over the developmental time of the nodule, a constitutive expression of the gene(s) of interest. Uses of said sequence, derived vectors and recombinant bacteria are also described.

## Description

The present invention relates to the use of the promintron DNA sequence of the *rolA* gene of *Agrobacterium rhizogenes* strain A4, or of promintron DNA sequences derived thereof, or of promintron DNA sequences of homologous genes from other strains of *Agrobacterium rhizogenes*, for promoting gene expression at the transcriptional level in recombinant bacteria and to achieve, for example, synthesis of phytohormones within bacteria and/or bacteroids.

The present invention demonstrates that the *iaaM* gene of *Pseudomonas syringae* and the *tms2* gene of *Agrobacterium tumefaciens* are able to improve the nodulation process and nitrogen fixation when expressed as bicistronic unit in *Rhizobia* under the Control of the promintron sequence. The promintron is particularly suited and useful for the use to drive gene expression within bacteroids in all stages of nodule development in order to obtain, over the developmental time of the nodule, a constitutive expression of the gene(s) of interest. Therefore, constitutive here means that, by different expression patterns at various stages of nodule development, a gene expression in all parts of the nodule where plant cells harbour *Rhizobia* is achieved.

The invention stems from the undisclosed demonstration that the 85 bp intron sequence of the *rolA* gene of *Agrobacteria* strain A4 is able to promote gene expression in prokaryotic cells. Therefore, the 85 bp DNA sequence has been named "promintron", indicating its function of intron in eucaryotic cells (Magrelli, A. et al. (1994) Science, 266, 1986-1988), and of promoter in prokaryotic cells. The term "promoter" refers to nucleotide sequences necessary for transcription initiation, i.e. RNA polymerase binding, and also includes for example the -35 and -10 boxes, or other regulatory regions.

The promoter activity in *Rhizobia* of the *rolA* promintron resulted to be able to get gene expression in a novel and peculiar pattern within root nodules, resulting in a constitutive expression.

The invention is particularly useful: i) to alter nodule development and to increase nitrogen fixation by either nodulating plants and/or by nitrogen fixing bacteria; ii) to improve plant growth and to accelerate plant biomass production by inoculating leguminous plants with genetically modified *Rhizobia* (*Rhizobia* is here used as a collective name including *Rhizobium*, *Sinorhizobium*, *Azorhizobium*, *Mesorhizobium* and *Bradyrhizobium*).

The present invention also relates to DNA constructs in which said promintron controls the expression of a DNA sequence, which upon expression leads to the above mentioned effects. Furthermore, the present invention relates to genetically engineered bacteria which can improve nitrogen fixation and consequently, plant biomass production. Furthermore, the improvement of nitrogen fixation in plants might be achieved in the presence of concentrations of nitrate negatively affecting the nodulation process.

The world population will most likely increase of 2 billions people in the next 25 years mostly in Asia and Africa. Proteins coming from legumes represent actually 33% of total human diet. Legume plants are able to self provide nitrogen through associations with nitrogen fixing bacteria of the genus *Rhizobia*, i.e *Rhizobium*, *Sinorhizobium*, *Azorhizobium*, *Mesorhizobium* and *Bradyrhizobium*. Other plants do exist not belonging to the *Leguminosae*, and yet able to host bacteria in a nitrogen-fixing nodule. A plant not belonging to the leguminous family interacting with nitrogen fixing *Rhizobia* is *Parasponia* (Ulmaceae). Furthermore, actinomycetes of the genus *Frankia* interact with plant species from eight different families and cause the production of root nodules (Pawlowski, K. et al. (1998) in: Biological Nitrogen Fixation for the 21^{st} Century, eds. C. Elmerich, A. Kondorosi and W.E. Newton, Kluwer Academic publisher, pp.199-201). Symbiotic nitrogen fixation plays a major role in the nitrogen cycle. Nevertheless, agriculture exploitation results in a large uptake of organic nitrogen from the soil, which is compensated by providing plants with fertilisers. Nitrogen fertilisation however, provokes severe environmental problems because its excess contributes to water pollution. Nitrogen fertilisation represents one of the most important part of the farming costs. The world consumption of nitrogen fertilisers increased in the last 55 years from 3 to 100 TG (1TG=10¹²g). To produce nitrogen fertilisers, all over the world 2 to 5% of the not renewable energy sources (e.g. oil and other forms of fuel) are burned every year. An environmental problem associated with the use of synthetic nitrogen fertilisers is leakage of excess nitrate into the underground water. Up to 10% of the released nitrate ends as nitrous oxide, a greenhouse gas whose energy reflectivity amounts to 180 times that of carbon dioxide (Hardy, R.W.F. and Eaglesham, A.R.J. (1995). In: Nitrogen Fixation: Fundamentals and applications. I.A. Tikhonovich et al., (eds.), pp. 619-620). Many large towns in industrialised countries are presently facing the problem of nitrate pollution of drinkable water, and water price for human use will rapidly increase.

Therefore, the general problem underlying the present invention is represented by the need to improve the cultivation of leguminous and other nodulating plants, with the aim to contribute to a drastic decrease of the nitrate release in the environment. A solution to these problems is either to increase the cultivation of plants able to fix atmospheric nitrogen or to increase their nitrogen fixation capacity.

Biological Nitrogen Fixation (BNF) by free-living, associative and symbiotic nitrogen-fixing microbes is responsible for the conversion of about 120 million tons of atmospheric nitrogen into ammonia every year, which represent 40% of the total nitrogen request (Vance, C.P., Third European Nitrogen Fixation Conference, Lunteren, The Netherlands, 1998, p. 17); symbiotic nitrogen fixation is the major naturally occurring mechanism by which nitrogen is reduced (Franssen, H.J. *et al*. (1992) Plant Molec. Biol., 19, 89-107). Members of the *Rhizobiaceae* family, which includes nitrogen-fixing *Rhizobia*, are able to enter into symbiosis with mainly leguminous plants, resulting in the formation of root nodules. Within the cells of the root nodule, the bacteria differentiate in bacteroids. Mature bacteroids fix atmospheric nitrogen and convert it into ammonia by a nitrogenase activity. The so-produced ammonium is exported from the nodules and it is incorporated into amino acids and proteins in the plant. Thus, in the presence of a nodulated root, plant growth is independent of alternative nitrogen sources.

Root nodule development is the result of a symbiotic interaction controlled by several factors. Early phases of this interaction require the exchange of chemical signals between the two partners: the plant root and the bacteria. The plant root produces chemical signals, such as flavonoids, which attract the *Rhizobia*, and most importantly do induce the expression of bacterial genes, the *nod* genes, involved in the production of the so-called Nod factors. The Nod factors are lipooligosaccarides which play a crucial role in the early phases of nodule development (Denariè, J., Debellè, F., and Promè, J.-C. (1996) Annual Review Biochemistry 65, 503-535) .

While the role of the Nod factors in triggering root nodule development is well established, the role of phytohormones in root nodule development is still unclear. Phytohormones are plant growth factors able to modify plant growth and development. Since phytohormones are involved in almost any developmental and/or physiological process of the plant, a role of phytohormones in nodule development is most reasonable. However, despite efforts aimed to address the general question of a role of phytohormones in nodulation, in 1994 Hirsch and Fang summarised the state of the art with the following words: "Important questions remains unanswered: *which* hormones take part in nodule formation, and *how* are they involved?" (A.M. Hirsch and Y. Fang, (1994) Plant Mol. Biol. 26, 5-9).

Already in 1936, Thimann (Thimann, K. (1936) P.N.A.S. USA, 22, 511-514) proposed that auxin(s) plays a role in nodule development, and that root nodules do contain auxin(s). Furthermore, based on biological assays, Thimann showed that the auxin content, estimated as auxinic biological activity, within a nodule is higher than that one of uninfected roots.

Nowadays, it is well established that free living *Rhizobia* do contain auxins, such as indole-3-acetic acid (IAA), indole-3-ethanol, indole-3-aldehyde and indole-3-methanol (Ernstsen, A. et al. (1987) Planta 171, 422-428). Furthermore, flavonoids, i.e. chemical signals produced by the plant root which attract *Rhizobia* and stimulate the synthesis of Nod factors, stimulate IAA synthesis in *Rhizobia* grown in liquid culture (Prinsen, E. et al. (1991) FEBS Letters, 282, 53-55).

Although these data are consistent with a possible role of auxin(s) in nodule development, Hirsch in 1992 reported that "*Rhizobia* can synthesize plant hormones, but mutations in their auxin-producing genes do not prevent nodulation, nor do Nod- mutants fail to produce hormones (Hirsch, A.M. (1992) New Phytologist, 122, 211-237).

Other studies addressing the "plant contribution" to the nodulation process, such as those of Hirsch, A.M. et al. (P.N.A.S. USA (1989) 86, 1244-1248), of Allen, E.K., Allen, O.N. and Newman, A.S. (Am. J. Botany (1953), 40, 429-435), and of Torrey, J.G. (in: "New root formation in plants and cuttings", 1986 ed. Jackson MB, Martinus Nijhoff Publishers, Dordrecht, 31-66), have found that inhibitors of polar auxin transport induced pseudonodules on alfaalfa roots. Mathesius et al. (The Plant Journal (1998) 14, 23-34) has recently shown that auxin transport inhibition precedes root nodule formation. Kijne, J.W. et al. (Third European Nitrogen fixation Conference, Lunteren, The Netherlands, 1998, p. 24), have shown that: i) a local increase of IAA precedes induction of cell divisions in the inner cortex and ii) nitrate inhibits the response to IAA rather than its accumulation.

Further indications of a role of auxinic phytohormones produced by the plant tissue in nodule development come from variants of *Medicago varia*, defined as auxin sensitive and usually containing a higher content of auxins, which formed more nodules than less sensitive variants (Kondorosi, E. et al. (1993) In: Nester, E.W. and Verma, D.P.S. eds., Advances in Molecular-Genetics of Plant microbe interactions, pp. 143-150) . Furthermore, plants transgenic for the *rolB* gene nodulated faster and had more nodules (Kondorosi et al., ibidem). RolB is known to increase auxin sensitivity. The function of rolB is considered by some authors not to affect auxin content and to be a membrane receptor for the auxin IAA, whereas others (PCT Application No. WO 98/28430) consider the RolB protein a beta-glucosidase able to release the auxin indolethanol from indolethanol-glucoside. Indolethanol is considered to be an auxin per se, and it is converted to IAA, the major form of auxin in plants (Sembdner, G. et al. (1980) in: Encyclopedia of Plant Physiology, vol. 9: Hormonal regulation of development. I. Molecular aspects of plant hormones, pp. 281-444; MacMillan, J. Ed., Springer, Berlin, Heidelberg, New York; Sandberg, G., Crozier, A., Ernstsen, A. (1987) In: The Principles and practices of plant hormone analysis, vol. 2, pp. 169-301, Rivier, L., Crozier, A. eds. Academic Press, London).

The present invention has addressed three aspects related to the aforementioned problems: i) is it possible to modify root nodule development by expressing auxin synthesising genes within *Rhizobia*?; ii) has the plant nodulated by the genetically modified strain of *Rhizobia* an increased capacity to reduce atmospheric nitrogen to ammonia? iii) is the plant nodulated by the genetically modified *Rhizobia* altered in its growth, and is such alteration improving plant biomass production?

Genes coding for enzymes able to modify auxin metabolism can be taken from the genomes of plants, but also from the genomes of bacteria (Spena, A., Estruch, J.J. and Schell, J. (1992) Current Opinion in Biotechnology 3, 159-163) . Whatever the origin of the gene(s) and/or coding region(s), the proper specificity of expression within the bacteria and, most importantly, within the bacteroids must be conferred to the said coding region(s) by positioning the coding region(s) under the control of a proper promoter (DNA) sequence.

The present invention solves the problems of the prior art by providing technical means to improve gene expression in recombinant bacteria, preferably *Rhizobiaceae*, and most preferably *Rhizobium*, and it is easily utilised to properly control the expression of genes able to increase the auxin content and, as consequence, to improve the nodulation and nitrogen fixation processes. The promintron sequence of the invention is able to promote prokaryotic expression in all the zones of the nodule where bacteroids are located with a temporally defined pattern of expression. Furthermore, the promintron drives gene expression during the exponential phase of growth and it is induced during the stationary phase of growth of free living bacteria. All these features are very advantageous when gene expression is to be promoted in nitrogen fixing soil bacteria under both exponential and stationary phase of growth, and in bacteroids within the root nodule.

In particular a gene construct of the invention comprises the promintron sequence of the *rolA* gene, as in SEQ ID No.1, and the coding regions of the *iaaM* from *Pseudomonas syringae* subsp. *savastanoi* and the *tms2* gene from *Agrobacterium tumefaciens*. The two coding regions have been built as bicistronic unit under the control of the *rolA* promintron, whose promoter activity takes place in the nodule in an undisclosed and novel pattern of expression. The combination of the promintron to the bicistronic unit, and its introduction in *Rhizobia*, has allowed the authors of the invention to show that auxin affects nodule development and function. The use of the two coding regions is meant to increase the synthesis and content of IAA, and consequently to increase also the synthesis and content of IAA conjugates (collective name indicating all the forms of IAA covalently conjugated either to amino acids or to glucose or to other chemical moieties) in the plant cells of the root nodule. The same effect might be achieved either by using the *iaaM* and *tms2* genes from other *Agrobacteria* or *Pseudomonas* strains, or by using other genes able to increase IAA content.

Since *Rhizobia* usually do not contain indoleacetamide, neither are able to convert tryptophane to indoleacetamide (IAM), nor are able to convert IAM to IAA (Ernstsen et al., 1987, ibidem), the authors have engineered a novel biochemical pathway which requires both genes to efficiently synthesise IAA from tryptophane. However, in those *Rhizobia*, such as *Bradyrhizobium*, which do contain the *bam* gene, an endogenous homologue of the *tms2* gene (Sekine, M., Watanabe, K. and Syono, K. (1989) J. Bacteriol. 171, 1718-1724), it might be sufficient to express only the *iaaM* gene under the control of the promintron DNA sequence.

In a further embodiment of the present invention the DNA sequence expression of which is driven by the promintron is one coding for indolepyruvate decarboxylase. Indolepyruvate decarboxilase is the key enzyme in the indolepyruvic pathway of IAA synthesis in which IAA is synthesised from tryptophane. The conversion of indolepyruvic acid to IAA is the rate limiting step of this pathway. A DNA sequence encoding a suitable indolepyruvate decarboxylase has been cloned, for example, from *Enterobacter cloacae* (see, e.g. Koga et al., Mol. Gen.Genet. (1991), 226, 10-16).

The term "auxin" comprises in this context naturally occurring and synthetic organic substances acting as phytohormones in the sense that they promote elongation of shoots and inhibit elongation of roots, preferably in low concentrations, most preferably already in concentrations lower than 10⁻⁶ M. Preferably, an auxin shows at least one of the following effects on plant development: stimulation of cell division, of cell elongation, and/or of cell expansion, of apical dominance, stimulation of xylem differentiation, stimulation of the cell elongation and cell division activity of the cells of the cambium, stimulation of lateral and adventitious root formation, stimulation of nodulation, of germination, of leaf epinasty, of ovary cell growth, of parthenocarpy, of the formation of female flowers and of leaf expansion. More particularly, the term "auxin" refers to indole 3-acetic acid (IAA) which is most likely synthesised in plant cells from tryptophane via indole-3-pyruvate and indole-3-acetaldehyde, and which is degraded via enzymatically catalysed oxidation. However, the term also comprises other naturally occurring compounds which act as an auxin and which are derived from indole or another compound, for example, the naturally occurring phenyl acetic acid which is a non-indolic auxin or 4-(indole-3-yl) butyrric acid. Furthermore, this term comprises compounds from organisms other than plants or chemically synthesised compounds which have at least one of the effects on plant development as listed above. An example for such compound is (2,4-dichlorophenoxy)-acetic acid (2,4-D).

In a preferred embodiment the DNA sequence linked to the promintron codes for a polypeptide which is naturally involved in the biosynthesis of at least one auxin. The expression of the DNA sequence in *Rhizobia* cells leads to an increase in the biological (for example, enzymatic) activity of this polypeptide and consequently to the increase of the content of at least one auxin first in the bacteroid, and after auxin export from the bacteroids, in plant cells of the nodule. Thus, in principle, by this embodiment it is contemplated that the auxin content and/or activity can be increased by increasing the biosynthesis of at least one auxin due to a stimulation/acceleration of a biosynthetic pathway leading to the synthesis of auxin. In another preferred embodiment the DNA sequence linked to the promintron codes for a protein which is naturally not expressed in *Rhizobia* and which upon expression in bacteroids leads to the synthesis of at least one auxin or a precursor of an auxin from a metabolite present in the bacterial cell and/or bacteroid. One example of a gene which is preferably used for the purpose of the present invention is the *iaaM* gene of *Pseudomonas syringae* subsp. *Savastanoi*, the etiological agent of plant tumors in olive and oleander trees (Spena, A., Estruch, J.J., Schell, J., Curr. Opinion in Biotechnology (1992), 3, 159-163). The neoplastic development is caused by phytohormones synthesised by the bacteria, which are then secreted into the surrounding tissues and stimulate the localised growth of plant cells. Among the genes involved in the pathogenesis of this type of tumour, the *iaaM* gene codes for the indoleacetamide monooxigenase, and it is responsible for converting by oxidation the amino acid tryptophane to indoleacetamide. Indoleacetamide has no particular auxin activity, but it is converted to IAA by the hydrolase encoded by the *iaaH* gene of *Pseudomonas syringae* subsp. *Savastanoi*, which is homologue to the *tms2* gene of *Agrobacterium tumefaciens*.

The *iaaM* gene from *Pseudomonas syringae* subsp. *Savastanoi* is known and its sequence has been published (Yamada et al. (1985) P.N.A.S. USA, 82, 6522-6526). The *tms2* gene from *Agrobacterium tumefaciens* is known and its sequence has been published (Klee, H. et al. (1984) P.N.A.S. USA 81, 1728-1732). According to the invention genes homologous in function to the *iaaM* gene of *P. syringae* and to the *tms2* gene of *Agrobacteriu*m *tumefaciens* might be used for the purpose of this invention. Such genes which are preferably also homologous with respect to the nucleotide sequence can be isolated by the person skilled in the art using known methods, e.g. the screening of cDNA or genomic libraries with probes designed on the basis of the *iaaM* gene of *Pseudomonas syringae* and/or *tms2* gene of *Agrobacterium tumefaciens*. Such genes with an activity similar to that of the *iaaM* gene product of *P. syringae* have been cloned, for instance, from some strains of *Agrobacteria* (i.e. *Agrobacterium tumefaciens* and *rhizogenes*; see, for instance: Klee et al. (1987), Gene Dev, 1,186-196; White et al. (1985) J. Bacteriol., 164, 33-44; Cardarelli et al. (1987), Mol. Gen. Genet., 208, 457-463.

The 85 bp DNA promintron sequence of the *rolA* gene from Ri plasmid A4 (SEQ ID No.1) promotes gene expression of DNA sequences positioned under its control (i.e. linked to its 3' end) in prokaryotic systems, such as *E.coli*, *Rhizobia* and *Agrobacteria*, during exponential, post-exponential and stationary phases of growth. Furthermore, the promintron promotes gene expression in bacteroids within root nodules. The person skilled in the art may easily and with no inventive effort isolate promintron sequences from other strains of *Agrobacterium rhizogenes* and/or isolate functional homologue promoters, namely promoters driving gene expression with an identical pattern in bacteroids within root nodules and/or in free living bacteria.

It is therefore a specific object of the present invention the use of the promintron sequence of the *rolA* gene from *Agrobacterium rhizogenes* as in SEQ ID No. 1, or of DNA sequences comprising said promintron sequence, or of functional homologue or portion thereof, to induce the expression of a DNA coding sequence, in recombinant bacteria during exponential, post-exponential and stationary phase of growth, and in bacteroids within root nodules, said coding DNA sequence being under the control of said promintron sequence, or of functional homologous promoters. Preferably the recombinant bacteria belong to either the *Enterobacteriaceae* or the *Rhizobiaceae* families, more preferably are *E. coli*, *Rhizobia* or *Agrobacteria*.

In a preferred embodiment the recombinant bacteria are of the *Rhizobia* genus, either within symbiotic root nodules or in a free living status. When bacteria are within symbiotic root nodules, they are either bacteroids of stage I, II, III, IV, V, or *Rhizobia* present in the apoplastic space, or *Rhizobia* present in the senescence zone, or *Rhizobia* present in the nitrogen fixing zone, or *Rhizobia* present in the invasion zone.

It is further object of the invention a recombinant DNA molecule comprising the promintron sequence according to the invention, or functional homologue or portion thereof, and covalently linked to the 3' end of the promintron sequence, a DNA coding sequence, wherein the recombinant DNA molecule is either harboured by episomal elements or integrated in the bacterial genome. Preferably the DNA coding sequence is either a monocistronic or a polycistronic transcription unit, more preferably encodes a protein involved in plant hormone synthesis and/or metabolism, most preferably encodes a protein involved in plant hormone auxin synthesis and/or metabolism, most preferably encodes a protein involved in the synthesis and/or metabolism of the auxin IAA or of the auxin indolethanol, even more preferably encodes the iaaM protein from *P. syringae subsp*. *savastanoi* or an homologous thereof and/or encodes the tms2 protein from *A. tumefaciens* or an homologous thereof. In a preferred aspect the recombinant DNA molecule according to the invention comprise both the *iaaM* and the *tms2* coding regions.

Alternatively the recombinant DNA molecule of the invention comprises a DNA coding sequence encoding the indolepyruvate decarboxylase from *Enterobacter cloacae* or an homologous thereof.

It is a further object of the invention genetically engineered bacteria comprising the recombinant DNA molecule described.

It is in the scope of the invention the use of the recombinant DNA molecule to increase nodule size and/or activity, and/or to increase plant biomass production.

It is in the scope of the invention the use of the recombinant DNA molecule to increase the capacity of nitrogen fixation of root nodules.

The invention will be described in some examples by reference to the following figures:

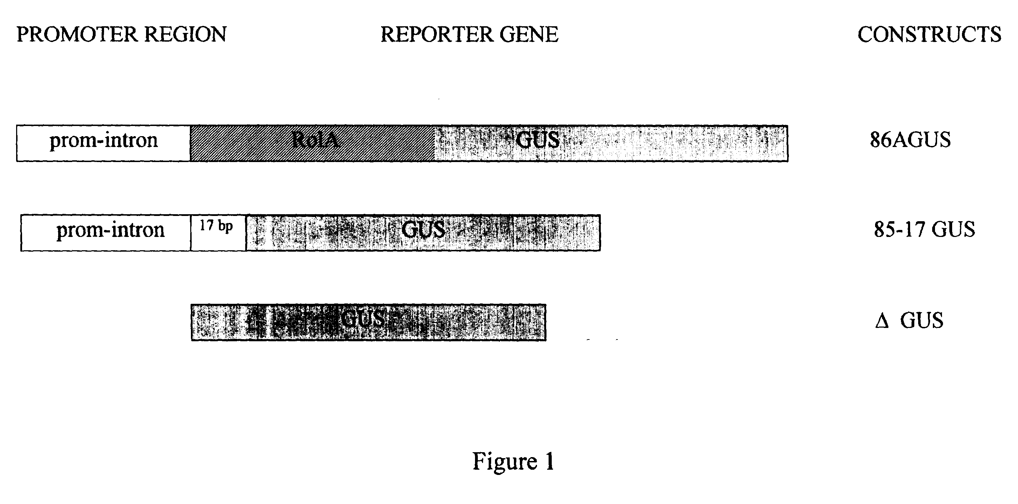

Figure 1: Schematic drawing of the chimeric reporter genes. The 86AGUS construct contains: i) an 86 bp long DNA fragment comprising the 85 bp long promintron sequence as shown in SEQ ID No. 1 plus one A residue at its 3' end; ii) the DNA sequence corresponding to the first 40 amino acids of the rolA protein fused to iii) the *uidA* coding region. The 85-17GUS construct contains 5' to 3' end: an Eco RI restriction site (GAATTC), the promintron sequence of SEQ ID No. 1, a 17 bp linker sequence (SEQ ID No. 2), a KpnI restriction site (GGTACC), before the ATG of the *uidA* coding region. Δ GUS indicates the construct containing just the *uidA* coding region, i.e. without any promintron sequence. Promintron indicates the 86 bp sequence comprising the 85 bp of the *rolA* promintron (from -1 to -86 being +1 the A of the ATG initiation codon of the *rolA* gene). *RolA* coding region indicates the sequence coding for the NH₂ terminal 40 amino acids of the rolA protein. GUS indicates the *uidA* coding region. The constructs were cloned in both orientation, as EcoRI fragments, in the vector pG, a derivative of pMB393 (Gage, D.J., Bobo, T. and Long, S.R. (1996) J. Bacteriology, 178, 7159-7166, see materials and methods).

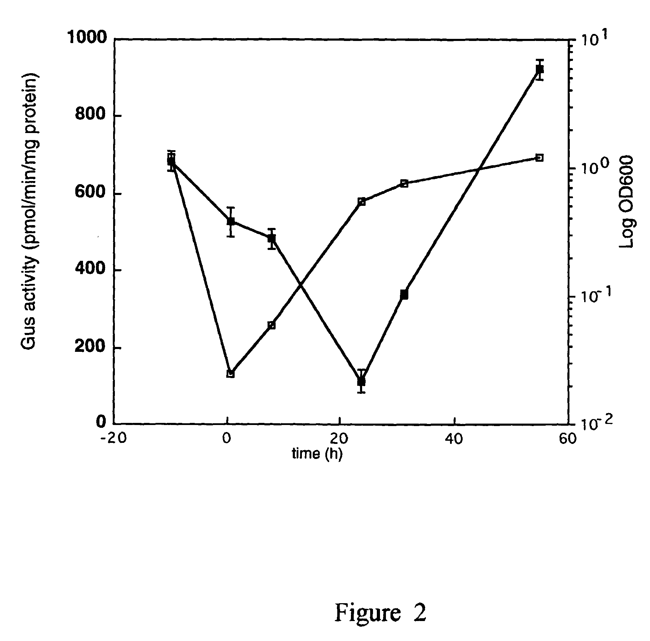

Figure 2. Growth phase dependent expression of β-glucuronidase activity in *Rhizobium leguminosarum* containing the 86AGUS construct. Cells were grown in rich media (Tryptone-Yeast Extract rich media) until stationary phase (first sample on the negative side of the temporal axis), then diluted one hundred folds in TYR media. The optical density at 600 nm (open squares) and specific β-glucuronidase activity (closed squares) are shown. The values are means of at least four independent experiments. SE means standard error. The construct Δ GUS, containing just the *uidA* (GUS) coding region, showed undetectable levels of specific β-glucuronidase activity and consequently has not been represented in the figure.

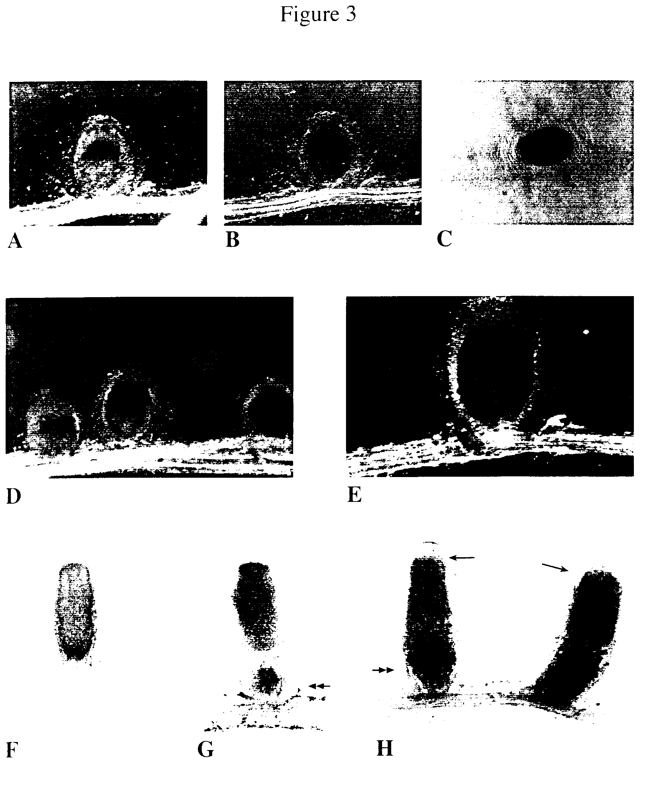

Figure 3. Root nodules from *Vicia hirsuta* plants infected with *R.l. viciae* containing the 86AGUS construct were stained for β-glucuronidase activity at different age. Panel A to D show one week old nodules. Panel A shows only few layers of cells infected, and consequently (blue)stained, just behind the meristem (indicated with an asterisk in panel E) . Panel B shows that in this developmental stage the infection zone is enlarging and will occupy later (Panel C) the whole nodule. Panel D shows three nodules at three different stages of infection on the same root. Panel E shows a two weeks old nodule; note the cylindrical shape of the nodule where all the inner tissue is invaded by bacteroids expressing GUS activity. Panel F shows a three weeks old nitrogen fixing nodule where Zone II and III are (blue) stained while the senescent Zone IV is mostly not stained. Panel G shows a four weeks old nodule displaying an intense staining in the late senescent Zone closest to the root. Panel H shows a double staining for GUS activity and for starch deposition (see Materials and Methods); Note the interzone II-III indicated by the arrows. Above the interzone II-III is visible an invasion Zone II reduced in size and yet still (blue) stained; the double arrow shows (blue) spots of GUS activity closest to the root.

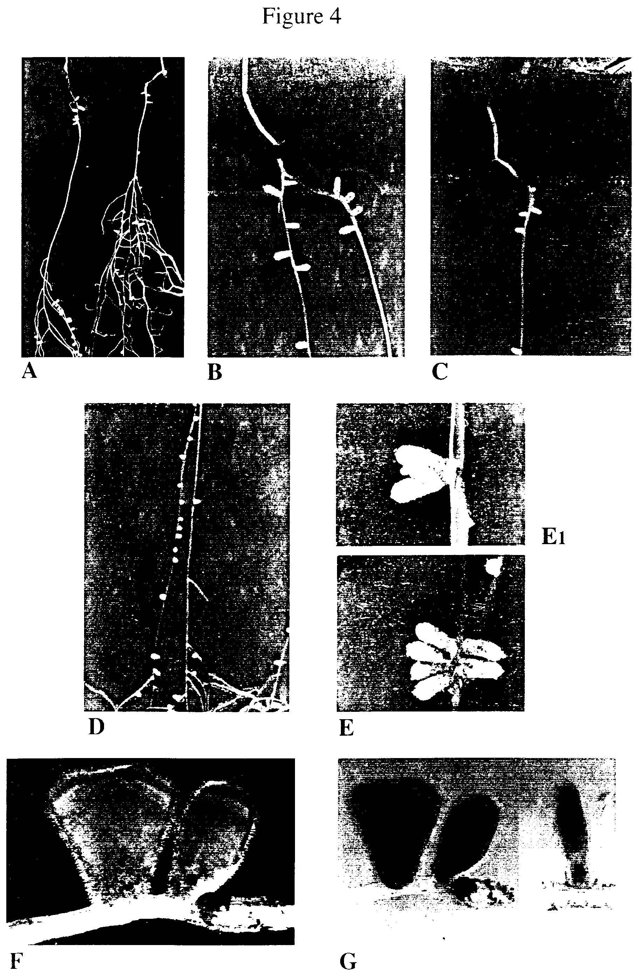

Figure 4. Panels A to E1: roots infected with wild type *R.l. viciae* containing either the 86AGUS construct (in panels C, D and on the right side of panels A and G) or the promintron-*iaaM-tms2* construct (panels B, E, E1, F and on the left side of panels A and G). Panels F and G are microscopic bright field images. Note that the root on the left side of panel A has been infected with bacteria containing the promintron-*iaaM-tms2* construct and has bigger nodules, clustered close to the seed and followed by a long region of the root without any nodule. The most distal part of the root has again nodules. The root on the right side of panel A has been infected with *R.l. viciae* containing the 86AGU3 construct: it shows a regular emersion of nodules all along the root (amplified in panel D). Panels B and C allow to compare the size of nodules elicited with wild type (panel C) and genetically modified *Rhizobia* containing the promintron-*iaaM-tms2* construct (panel B). Panel E and E1 show the morphology of two typical nodules elicited by bacteria harbouring the promintron-*iaaM-tms2* construct: bilobate in panel E or highly clustered in panel E1. In panel F is shown a dark field microscopic view of a clarified (see Materials and Methods) bilobate nodule derived from inoculation with *R.l. viciae* containing the promintron-*iaaM-tms2* construct. In panel G the same couple of nodules shown in panel F is compared to a nodule of an identical age derived from a *R.l. viciae* harbouring the 86AGUS construct and stained for β-glucuronidase activity.

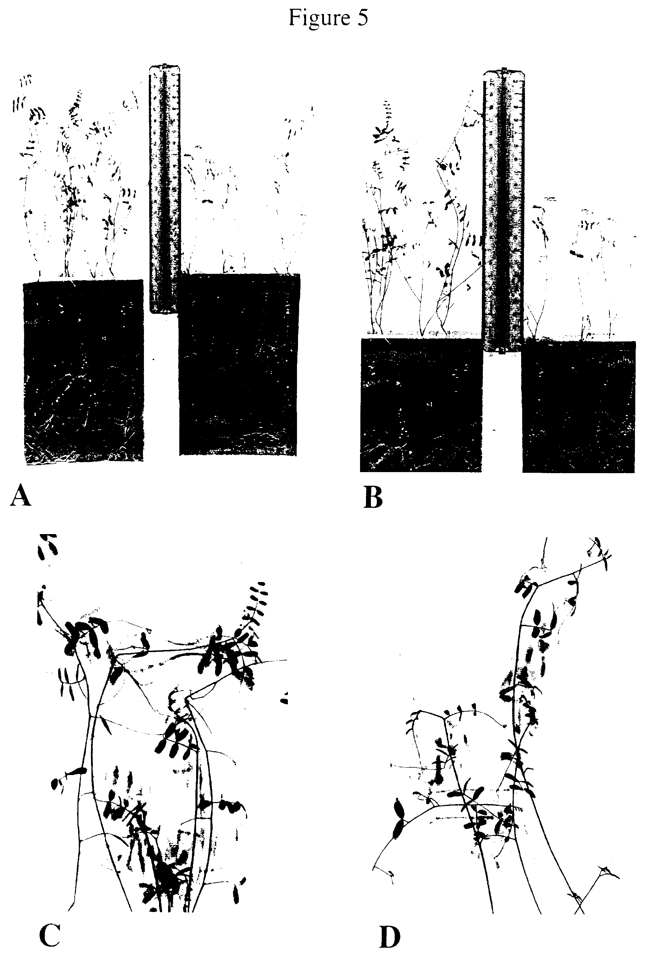

Figure 5. *Vicia hirsuta* plants were grown in seed-growth pouches (Mega International, Minneapolis), five seeds per pouch. On the left part of panels A and B, and in panel C are shown 35 days old plants infected with *R.l. viciae* harbouring the promintron-*iaaM-tms2* construct; on the right part of panels A and B, and in panel D are shown plants infected with *R.l. viciae* containing the 86AGUS construct. Note in panels A and B the bigger size of plants infected with the promintron-*iaaM-tms2* construct in comparison to that one of plants infected with *Rhizobia* harbouring the 86AGUS construct. Plants nodulated by *R.l. viciae* containing the 86AGUS construct have (panel D) smaller leaves, less branches and more senescent leaves per plant. Leaves with a more dark green colour are displayed by plants nodulated by *Rhizobia* harbouring the promintron-*iaaM-tms2* construct (panel C), furthermore the plants do have a more healthy appearance (compare panel C with panel D).

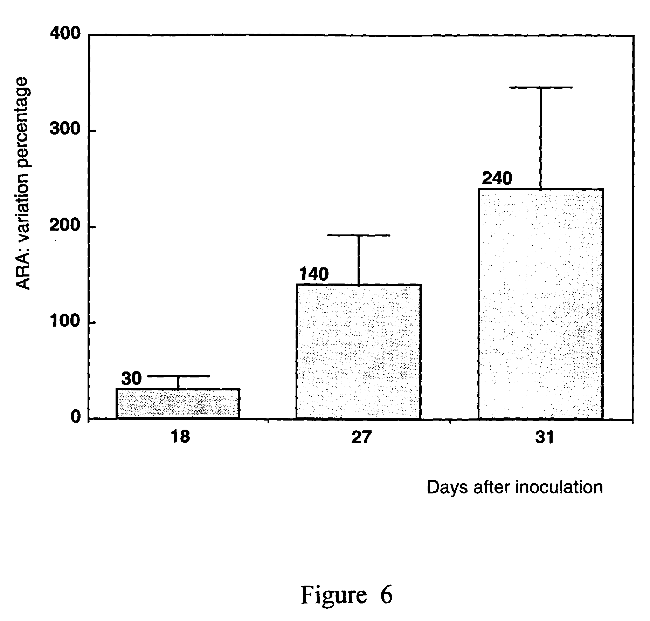

Figure 6. Acetylene reduction assays (ARA) . Data compare ARA of 18, 27 and 31 days old plants nodulated either with *R.l. viciae* harbouring the 86AGUS construct (control) or with *Rhizobia* harbouring the promintron-*iaaM-tms2* construct. In each case the control is considered as 100% and data indicate the percentage of increase of ARA obtained from roots nodulated with *Rhizobia* containing the promintron-*iaaM-tms2* construct. Each column represents the medium value obtained from fifty plants. ARA has been expressed in arbitrary units.

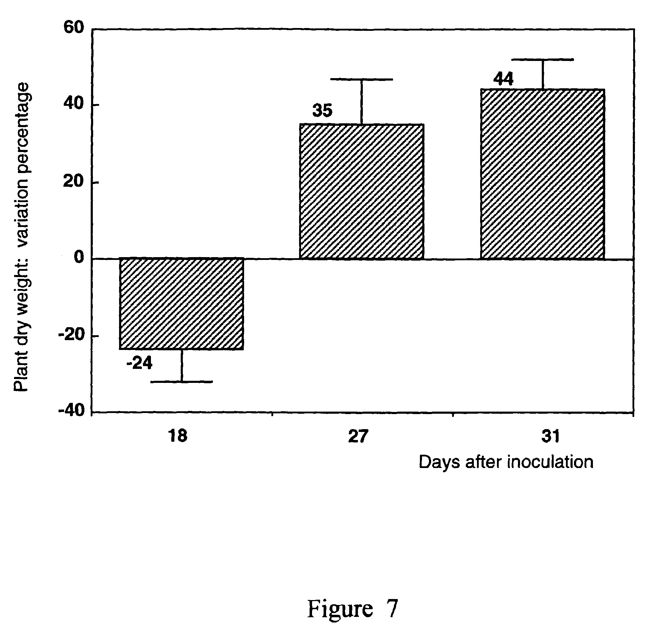

Figure 7. Plant dry weight. The stem of nodulated plants was cut just above the seed and dried in an oven at 85°C overnight. Panel A shows the plant dry weight of 18, 27 and 31 days old plants nodulated with *R.l. viciae* harbouring the 86AGUS construct (control) and those infected with *Rhizobia* containing the promintron-*iaaM-tms2* construct. In each case the control is considered as 100% and indicated as zero on the abscissa axe; data indicate the percentage variation, from the corresponding control, of dry weight obtained from stems of plants nodulated with the promintron-*iaaM-tms2* construct. Each column represents the value from 50 plants.

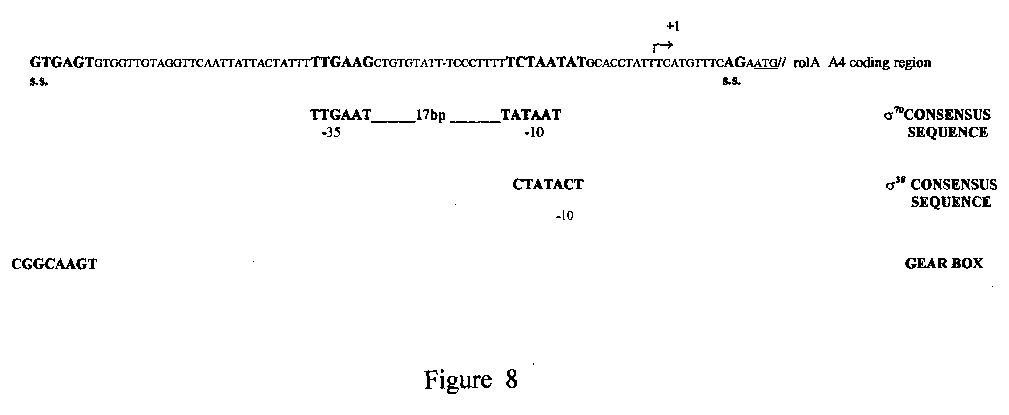

Figure 8. Nucleotide sequence of the *rolA* promintron from Ri plasmid A4 of *Agrobacterium rhizogenes*. Sequences showing homology to: i) the -10 and -35 regions of sigma70-dependent promoters, ii) -10 region of sigma38-dependent promoters, and iii) so-called GEAR-BOX sequence are printed in bold. The arrow indicates the main initiation of transcription.

Materials and Methods used are herein depicted:

### Construction of recombinant plasmids

Standard techniques were used for the construction of recombinant DNA plasmids. The 86AGUS, 85-17GUS and ΔGUS constructs were subcloned in both orientations as EcoRI fragments in the plasmid vector pG, a derivative of pMB393 (Gage, D.J., Bobo, T. and Long, S.R. (1996), ibidem) obtained by deletion of a 750 bp long EcoRI fragment, and then introduced by electroporation into *Rhizobium leguminosarum* biovar *viciae*, strain LPR1105, a rifampicin resistant derivative of RCR1001 (Hooykas P.J.J. *et al*. (1977) J. Gen. Microbiol., 98, 477-484). The construct pG-promintron-*iaaM-tms2*, comprising the *rolA* promintron driving the *iaaM* and *tms2* genes is described in Example 3.

### Growth conditions and GUS assay

*Rhizobium l*. b. *viciae* cells harbouring the different constructs were grown in TYR medium at 30°C. Growth curves were determined by measuring OD₆₀₀.

Bacteria, collected by centrifugation, were resuspended in 50mM Na-phosphate buffer (pH 7.0), 10mM β-mercaptoethanol, 10mM Na₂EDTA, 0.1% sodium lauryl sarcosine, 0.1% Triton X-100, sonicated for 5 min twice and centrifuged at 16.000 x g for 10 min at 4°C. GUS activity was assayed by using the supernatant. Fluorimetric GUS assays were performed as described by Jefferson (Jefferson, R. (1987) Plant Mol. Biol. Report, 5, 387-405) . Protein concentration in bacterial extracts was determined using the Bradford reagent (BIORAD) according to the manufacturer's instructions.

*Rhizobia* harbouring the different constructs were plated and selected on TYR rich media supplemented with 100 µg/ml spectinomicin, and replicated on plates containing 100 µg/ml rifampicin. Strains to be inoculated on plants were grown until stationary phase, centrifuged 5' at 12.000g, washed in PBS buffer pH 7.4 and resuspended in the same buffer. Optical Density (OD) was measured at 600 nm and 10⁵ cells were applied on each seedling and incubate for 1 hour. *Vicia hirsuta* seeds were surface sterilised in 5% hydrogen peroxide for 30' and then extensively washed with sterile bidistilled water. Seeds were allowed to swollen over-night at 4°C and then germinated on 1.5% agar plates for three days in the dark. After infection, germinated plantlets were grown in seed-germination pouches (MEGA International Minneapolis, USA) containing 10 ml of Jensen media (Vincent, J.M. (1970) I.B.P. Handbook No. 15, Oxford, Blackwell Scientific publications) . Plants were grown in 70% humidity, 16/8 light/dark period at 17 or 22°C.

### In situ GUS staining

Roots from nodulated plants were cut and incubated at 37°C overnight in X-Gluc Reagent mix (1mM X-Glucuronide, 0.1mM NaPO₄, 10mM EDTA pH7.0, 0.5mM K ferrocyanide pH 7.0, 0.5mM K ferrocyanide pH 7.0, 0.1% Triton X-100) then fixed in 4% paraformaldehyde in PBS buffer (GUS Protocols by Gallagher S.G., 1992, Academic Press, Inc.) . Roots were then dehydrated in increasing concentration of absolute ethanol and clarified by immersion in a mixture 2:1 benzyl benzoate-benzyl alcohol (Sigma) . For starch staining whole nodules were immersed for 20 seconds in an aqueous solution of 0.1M potassium iodide. Whole nodules were photographed with a Nikon microscope in bright-field and epipolarization optics.

Acetylene reduction assays (ARA) were performed by cutting nodulated roots (4 roots per 15 ml tube) and injecting 1 ml of acetylene for at least one hour at room temperature. Acetylene and ethylene peaks were obtained by injecting one millilitre from each tube into a Perkin Elmer Sigma 3B gas chromatograph equipped with a column Porapack Q equipped with a Perkin Elmer 561 recorder.

### Example 1: The spliceosomal intron of the rolA gene from Agrobacterium rhizogenes is a promoter active in Rhizobia.

The DNA sequence, hereafter called promintron, spanning the *rolA* intron (which is 85 bp long and it goes from position -1 up to position -86, being +1 the A of the ATG initiation codon of the *rolA* gene) is able to drive prokaryotic expression in *Rhizobia.* A reporter gene construct (86AGUS; Fig.1) was built by fusing the coding region of the *uidA* gene (Jefferson, 1987, ibidem) to a fragment comprising the DNA sequence coding for the NH₂-terminal 40 amino acids of the RolA protein preceded by the promintron (i.e. 85 bp of the *rolA* promintron (SEQ ID No.1) plus the A immediately preceding the ATG initiation codon of the rolA coding region). GUS activity was detected by fluorimetric assays in extracts from *Rhizobium leguminosarum* biovar *viciae* (*R. l. viciae*) harbouring the construct 86AGUS (Fig.2) . A similar pattern of expression (data not shown) has been obtained by using the 85-17GUS construct which comprises the promintron of SEQ ID No. 1 and a 17 bp linker (SEQ ID No. 2) and the *uidA* coding region (Fig. 1). The construct ΔGUS consisting of the *uidA* coding region by itself (Fig.1) gave no GUS activity in *Rhizobia* harbouring such construct (data not shown). *R. l. viciae* strain LPR 1105 harbouring either of the three constructs were grown in Trypton-Yeast Extract (rich media) up to stationary phase at an optical density of 1.8 at 600 nm, then diluted 100 times in the same media. Samples were taken in early, mid and late exponential phase of growth and then in early and late stationary phase of growth. GUS activity was detected only in protein extracts from *R. l. viciae* harbouring the 86AGUS (fig.2) or the 85-17GUS (data not shown) constructs while ΔGUS showed undetectable levels of β-glucuronidase activity, as untransformed *Rhizobia*. The data obtained show that, in *Rhizobia* harbouring the 86AGUS (or 85-17GUS constructs), β-glucuronidase activity declines during the exponential phase of growth and it increases in early stationary phase and it reaches the highest value at late stationary phase of growth (Fig.2). The same experiment was repeated diluting 100 times the stationary colture grown in rich media into a minimal defined media containing a concentration of mannitol, used as carbon source, limiting for growth. After the dilution, a long period of growth lag was observed followed by an exponential phase of growth and by a stationary phase of growth reached at an optical density of 0.6. This reduced O.D. is caused by the carbon limiting condition applied. Promoters of enteric bacteria regulated by the stationary sigma factor sigma S are specifically activated under these conditions. The promintron sequence harbours a sigma S-like consensus sequence (Fig.8) . The GUS activity in *Rhizobia* harbouring the 86AGUS construct was enhanced during the stationary phase of growth and the values reached were 2-times higher than those measured under stationary phase of growth reached in rich media as a consequence of the carbon starvation. Thus, gene expression driven by the promintron sequence is inversely correlated to growth rate and it is induced at the onset of the stationary phase of growth. An identical pattern of expression has been found utilising the 85-17GUS construct where the promintron sequence is separated from the *uidA* gene by a 17 base pairs polylinker shown in SEQ ID No. 2. Thus, the 85 bp long promintron has a promoter function in *Rhizobia*. Open squares represent the O.D. at 600 nm; filled squares represent GUS activity.

### Example 2: The promintron drives gene expression within nodules with a novel and unexpected pattern of expression.

*Rhizobium leguminosarum* biovar *vicia*e strain LPR 1105 harbouring the 86AGUS construct or the 85-17GUS or the ΔGUS, where used to infect *Vicia hirsuta* plants. The plantlets were grown under greenhouse conditions up to 60 days after planting, and at different times roots were cut, fixed under buffered paraformaldehyde and stained for in situ GUS activity (Jefferson, 1987, ibidem), after over-night incubation in the appropriate buffer, roots were dehydrated by immerging into increasing ethanol solutions followed by toluene clarification and observed under an optical bright field microscopy. DNA fragments comprising the promintron DNA sequence shown in SEQ ID No. 1 promotes expression in the nodules within symbiotic *Rhizobia* with a characteristic and novel pattern of expression resulting in a constitutive expression of the gene of interest. For constitutive expression it is meant that expression, although timed in a specific pattern, overall it takes place in all the zones of the nodule where plant cells do contain bacteroids. This expression pattern is strikingly different from that one shown by either sigma 54 or sigma 70-like regulated promoters, for comparison see Sharma S.B. and E.R. Signer (1990) *Gene Dev*., 4, 344-356.

*Rhizobium* bacteria when entering into the host plant are called bacteroids. Bacteroids are known to undergo 5 differentiation forms progressively older from stage 1 to 5. The different stages are described in Vasse, J., et al. (1990) J. Bacteriol., 172, pp. 4295-4306. In an indeterminate nodule such that of *Vicia hirsuta* the youngest bacteroids are located in the early infection Zone II, close to the tip meristem (which represents Zone I) outgrowing from the root, where bacteria are recently released from the infection threads crossing first the root hairs and then the cortical layers to reach finally the growing nodule. Much older bacteroids are located in the late infection Zone II, then in the nitrogen fixing Zone III and finally in the senescent Zone IV only ghost membrane of bacteroids are present. Expression driven by the promintron was observed in Zones II, III and IV of the nodule at any age including in the very old senescent zone in the region most proximal to the root (see Figure 3). Thus, all plant cells harbouring bacteroids at any stage do have β-glucuronidase activity within their bacteroids. The pictures are taken from nodules infected by *Rhizobia* containing the 86AGUS construct. An identical pattern of expression has been obtained utilising the 85-17GUS construct that gives only a slightly weaker signal. No staining has been observed with *Rhizobia* harbouring the construct ΔGUS. In Fig. 3, panel I, is presented a double staining for GUS (blue) activity and for starch (dark brown). In older nodules starch deposition occurs in Zones III and IV, but it is mostly used because its staining describes the interzone II-III (see arrow in Fig.3, panel I). The GUS staining is both above and below the interzone II-III.

### Example 3: Construction of plasmid pG-promintron-iaaM-tms2

The recombinant plasmid pG-promintron-*iaaM-tms2* was obtained by ligating the EcoRI-KpnI fragment comprising SEQ ID No. 1 and SEQ ID No. 2, spanning the *rolA* promintron sequence, within the pG plasmid (see materials and methods) cut with EcoRI-KpnI. Afterwards the plasmid pG-promintron was cut with KpnI-HindIII and the *iaaM* and *tms2* coding sequences were introduced as KpnI-SphI and SphI-HindIII fragments, respectively. The KpnI-SphI fragment of 1775 bp (2+53+1671+49) spans the coding region of the iaaM gene from *Pseudomonas syringae* subsp. *savastanoi*. The iaaM sequence has been characterised by Yamada et al (1985, ibidem) and the sequence used contains 1773 bp (53+1671+49) from the DraI site located 53 bp before the ATG initiation codon till the SphI site 46 bp after the TAA stop codon. The SphI-HindIII fragment of 1452 (22+13+1403+14) bp spanning the *tms2* coding region from *Agrobacterium tumefaciens* pTi A6 (Klee, H. et al. (1984) P.N.A.S. USA 81, pp. 1728-1732) contains 1403 bp of coding region preceded by 14 bp and followed by 5 bp belonging to the untranslated regions of the *tms2* gene.

Consequently, the plasmid pG-promintron-*iaaM--tms2* obtained possesses the following structural features:
- a promintron fragment comprising the 85 bp of the *rolA* promintron (SEQ ID No. 1), plus 17 bp of linker sequence (SEQ ID No. 2) and having an Eco RI adapter (sequence GAATTC) at its 5' end;
- 6 bp as KpnI site (sequence GGTACC) plus extra 2 bases added as linker;
- 53 bp of 5' untranslated sequence of the *iaaM* gene from *Pseudomonas syringae* subsp. *savastanoi*;
- 1671 bp of coding region of the *iaaM* gene from *Pseudomonas syringae* subsp. *savastanoi*;
- 49 bp (46+3 bp of stop codon) of 3' untranslated trailer sequence of the *iaaM* gene from *Pseudomonas syringae* subsp *savastanoi* ending with the SpHI site;
- 22 bp consisting of a polilinker sequence (CTGCAGGTCGACTCTAGAGGAT, SEQ ID No. 3);
- 13 bp of untranslated leader region of the tms2 gene (CCAACTCAGAGAG, SEQ ID No. 4);
- 1403 bp spanning the coding region of the *tms2* gene from *Agrobacterium tumefaciens* pTiA6;
- 14 bp at the 3' end including an HindIII site (sequence TAAACATCAAGCTT, SEQ ID No. 5) being TAA the stop codon, AC sequence at the 3' of the *tms2* coding region after the stop codon, and the rest being HindIII linker sequence (HindIII site being AAGCTT).

### Example 4: The promintron-iaaM-tms2 construct alters root nodule number, location and size.

*Vicia hirsuta* plantlets were harvested at 18 days after infection with either the 86AGUS construct or the promintron-*iaaM-tms2* construct. In the case of plants infected with *Rhizobia* harbouring the 86AGUS construct, an average number of 22 nodules per plant was observed while this number decreased at 7.5 nodules per plant in the case of plants infected with *Rhizobia* harbouring the promintron-*iaaM-tms2* construct. Considering the control 86AGUS infected plants as 100%, the presence of the bicistronic operon in *Rhizobia* reduces the nodule number by 65%. In contrast to this feature, the nodule size is strongly increased in plants infected with *Rhizobia* harbouring the promintron-*iaaM-tms2* construct. In Figure 4, panels B and C, the size of the nodules can be compared. Not only *Rhizobia* containing the promintron-*iaaM-tms2* construct induced nodules which were bigger in size, but sometimes the nodules were also bilobate (panel E) . In addition, the root nodules elicited by *Rhizobia* harbouring the promintron-*iaaM-tms2* construct, are localised mostly on the primary root (Fig.4, panel A left) and rarely on secondary roots as is the case with plants inoculated with *Rhizobia* harbouring either the 86AGUS construct or not harbouring any construct at all (Fig.4, panel A, right) . Finally, Fig. 4 (panel A, left) shows that nodules elicited by *Rhizobia* harbouring the promintron-*iaaM-tms2* construct are concentrated in the root region close to the seed, followed by a large zone of the root where no nodule did occur. This was not the pattern of nodulation elicited by wild type *Rhizobium*, which consisted of small nodules distributed uniformly all along the root (Fig.4, panel A right, and panel D).

### Example 5: The root nodules induced by Rhizobia harbouring the promintron-iaaM-tms2 construct shows an increased acetylene reduction activity (ARA)

Nodulated roots infected with wild type *Rhizobium leguminosarum* biovar *viciae* strain LPR1105 or the same strain harbouring either the 86AGUS or the promintron-*iaaM-tms2* construct were followed for one month after infection and assayed for the ability to reduce acetylene to ethylene. This is the most commonly used indirect enzymatic assay to evaluate the ability of the nitrogenase complex to reduce atmospheric nitrogen to ammonia. Eight independent experiments using up to fifty plants per point have been carried out at two different temperatures (17°C and 22°C) . Results show that the promintron-*iaaM-tms2* construct induces the formation (at 22°C) of root nodules able to reduce acetylene to ethylene more efficiently than the control root nodules generated either by the wild type *Rhizobia* or by *Rhizobia* harbouring either the 86AGUS or the 85-17GUS. Thus, the presence of the promintron-*iaaM-tms2* construct increases the capacity of nitrogen fixation of root nodules. Samples were taken at 18, 27 and 31 days after inoculation of germinated seeds with bacteria. Acetylene reduction activity (ARA) was followed at the Gas Cromatograph (see Materials and Methods) and expressed in arbitrary units. In Fig. 6 the media of the value of the control (at least 50 plants in each case) are taken as 100%. Each column indicate the percentage of increase compared to the controls of ARA from roots nodulated with the promintron-*iaaM-tms2* construct. In function of time, the percentage of ARA increases from 30%, at 18 days, up to 240% more than controls at 31 days.

### Example 6. Plant growth and biomass

As shown in Fig. 5, 35 days old *Vicia hirsuta* plants nodulated by *Rhizobia* containing the promintron-*iaaM-tms*2 construct are bigger in size (more than 20% increase). Furthermore, these plants are not only bigger, but also altered in their phenotype having dark green leaves and a better vigour in comparison to sister plants inoculated with the wild type *R.l. viciae*. In Figure 7 are listed the dry weights of plants 18, 27 and 31 days old. The dry weight of control plants is considered 100% at any age and indicated as zero. It could be observed a significant decrease (i.e. 24%) in the average dry weight of plants nodulated with *Rhizobia* containing the promintron-*iaaM-tms*2 construct at 18 days, when the influence on plant growth of seed storage proteins is still important. In contrast, 27 and 31 days old plants, when the nitrogen contained in the seeds is mostly exhausted, shows a 35% and 44%, respectively, increase in dry weight of plants nodulated with *Rhizobia* harbouring the promintron-*iaaM-tms2* construct in comparison to the dry weight of plants nodulated by the wild type strain of *Rhizobia* or of *R.l. viciae* containing the 86AGUs or the 85-17GUS constructs.

## Claims

1. Use of the promintron sequence of the *rolA* gene from *Agrobacterium rhizogenes* as in SEQ ID NO. 1, or of DNA sequences comprising said promintron sequence, or of functional homologous or portion thereof, to induce the expression of a DNA coding sequence, in recombinant bacteria during exponential, post-exponential and stationary phase of growth, and in bacteroids within root nodules, said coding DNA sequence being under the control of said promintron sequence.

2. Use of the promintron sequence according to claim 1 wherein said recombinant bacteria belong to either the *Enterobacteriaceae* or the *Rhizobiaceae* families.

3. Use of the promintron sequence according to claim 2 wherein said recombinant bacteria belonging to either the *Enterobacteriaceae* or the *Rhizobiaceae* families are *E. coli*, *Rhizobia* or *Agrobacteria*.

4. Use of the promintron sequence according to claim 3 wherein said recombinant bacteria are of the *Rhizobia* genus, either within symbiotic root nodules or in a free living status.

5. Use of the promintron sequence according to claim 4 wherein said recombinant bacteria of the *Rhizobia* genus within symbiotic root nodule, are either bacteroids of stage I, II, III, IV, V, or *Rhizobia* present in the apoplastic space, or *Rhizobia* present in the senescence zone, or *Rhizobia* present in the nitrogen fixing zone, or *Rhizobia* present in the invasion zone.

6. A recombinant DNA molecule comprising the promintron sequence according to claim 1, or functional homologous or portion thereof, and covalently linked to the 3' end of said promintron sequence, a DNA coding sequence, said recombinant DNA molecule being either harboured by episomal elements or integrated in the bacterial genome.

7. The recombinant DNA molecule according to claim 6 wherein said DNA coding sequence is either a monocistronic or a polycistronic transcriptional unit.

8. The recombinant DNA molecule according to claim 6 or 7 wherein said DNA coding sequence encodes a protein involved in plant hormone synthesis and/or metabolism.

9. The recombinant DNA molecule according to claim 8 wherein said DNA coding sequence encodes a protein involved in plant hormone auxin synthesis and/or metabolism.

10. The recombinant DNA molecule according to claim 8 wherein said DNA coding sequence encodes a protein involved in the synthesis and/or metabolism of the auxin IAA or of the auxin indolethanol.

11. The recombinant DNA molecule according to claim 8 wherein said DNA coding sequence encodes the iaaM protein from *P. syringae* subsp. *savastanoi* or an homologous thereof.

12. The recombinant DNA molecule according to claim 8 wherein said DNA coding sequence encodes the *tms2* protein from *A. tumefaciens* or an homologous thereof.

13. The recombinant DNA molecule according to claim 8 wherein said DNA coding sequence encodes both the *iaaM* and the *tms2* coding regions of claim 11 and 12, respectively.

14. The recombinant DNA molecule according to claim 8 wherein said DNA coding sequence encodes the indolepyruvate decarboxylase from *Enterobacter cloacae* or an homologous thereof.

15. Genetically engineered bacteria comprising the recombinant DNA molecule according to claims from 6 to 14.

16. Use of the recombinant DNA molecule according to claims from 6 to 14 to increase the size of nodules, and/or the nodule capacity to fix nitrogen, and/or to increase plant biomass production.

17. Use of the recombinant DNA molecule according to claims from 6 to 14 to increase nitrogen fixation.


SEQUENCE LISTING

(1) GENERAL INFORMATION:

(i) APPLICANT:

(A) NAME: G.IN.E.ST.R.A. S.C.ar.l.
(B) STREET: Piazza Caduti 20
(C) CITY: Verona
(E) COUNTRY: Italy
(F) POSTAL CODE (ZIP): 37100

(A) NAME: Consiglio Nazionale delle Ricerche
(B) STREET: Piazzale Aldo Moro 7
(C) CITY: Roma
(E) COUNTRY: Italy
(F) POSTAL CODE (ZIP): 00195

(ii) TITLE OF INVENTION: Method to control gene expression in
bacteria, namely Rhizobiaceae, to improve root nodule
development, nitrogen fixation and plant biomass
production

(iii) NUMBER OF SEQUENCES: 5

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 85 base pairs
(B) TYPE: nucleic acid

(C) STRANDEDNESS: single
(D) TOPOLOGY: linear

(xi) SEQUENCE DESCRIPTION: SEQ ID NO: 1:

GTGAGTGTGG TTGTAGGTTC AATTATTACT ATTTTTGAAG CTGTGTATTT CCCTTTTTCT
60
AATATGCACC TATTTCATGT TTCAG
85

(2) INFORMATION FOR SEQ ID NO: 2:

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO: 2:

GGGTAGGTCA GTCCCTT
17

(2) INFORMATION FOR SEQ ID NO: 3:

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO: 3:

CTGCAGGTCG ACTCTAGAGG AT
22

(2) INFORMATION FOR SEQ ID NO: 4:

(i) SEQUENCE CHARACTERISTICS:
(A) LENGTH: 13 base pairs
(B) TYPE: nucleic acid
(C) STRANDEDNESS: single
(D) TOPOLOGY: linear

(xi) SEQUENCE DESCRIPTION: SEQ ID NO: 4:

CCAACTCAGA GAG
13

(2) INFORMATION FOR SEQ ID NO: 5:

(i) SEQUENCE CHARACTERISTICS:
(A) LENGTH: 14 base pairs
(B) TYPE: nucleic acid
(C) STRANDEDNESS: single
(D) TOPOLOGY: linear

(xi) SEQUENCE DESCRIPTION: SEQ ID NO: 5:

TAAACATCAA GCTT
14